# EUROPEAN PATENT SPECIFICATION

(11) **EP 0 172 194 B1**
(45) Date of publication and mention of the grant of the patent: **19.01.1994**
(21) Application number: 85900939.1
(22) Date of filing: 07.02.1985
(51) Int. Cl.: C12N 15/70, C12P 21/02, C12N 15/26

(54) **CONTROL SYSTEMS FOR RECOMBINANT MANIPULATIONS**
KONTROLLSYSTEME FÜR REKOMBINANT-MANIPULATIONEN
SYSTEMES DE REGULATION POUR DES MANIPULATIONS RECOMBINANTES

(30) Priority: 08.02.1984 US 578133; 19.04.1984 US 602118; 08.06.1984 US 618499; 31.08.1984 US 646693; 24.12.1984 US 685312
(43) Date of publication of application: 26.02.1986
(73) Proprietor: CETUS ONCOLOGY CORPORATION, Emeryville California 94608 (US)
(72) Inventor: GELFAND, David, H., Oakland, CA 94611 (US); LAWYER, Francis, C., Oakland, CA 94611 (US); STOFFEL, Susanne, El Cerrito, CA 94530 (US)
(74) Representative: Bizley, Richard Edward
(86) International application number: US8500196
(87) International publication number: WO8503522

(56) References cited:
- EP-A- 36 776
- EP-A- 41 767
- EP-A- 68 740
- GB-A- 2 039 916
- GB-A- 2 071 671
- GB-A- 2 104 901
- US-A- 5 079 352
- NUCLEIC ACIDS RESEARCH, vol. 11, 1983; E.REMAUT et al., pp. 4677-4688#
- NATURE, vol. 292, 09 July 1981; H.SHIMATAKE et al., pp. 128-132#
- NATURE, vol. 302, 24 March 1983; T.TANIGUCHI et al., pp. 305-310#
- PROCEEDINGS OF THE NATL. ACADEMY OF SCIENCE USA, vol. 80, August 1983; R.T.FRALEY et al., pp. 4803-4807#
- NUCLEIC ACIDS RESEARCH, vol. 11, October 1983; K.R.KASTER et al., pp. 6895-6911#
- CELL, vol. 13, January 1978; K.BACKMAN et al., pp. 65-71#
- Nucl.Ac.Res. 8 (1980) 3895
- Biotechnology Jan (1984) 81-85
- Gene 15 (1981) 81-93

## Description

The present invention relates to aspects of recombinant DNA technology concerned with providing control systems for expression. More particularly, the invention relates to a convenient temperature sensitive control cassette.

### Background Art

Alteration of the genetic makeup of a host cell is the modus operandus of current biotechnology. By suitable modification, such host cells may be caused to produce protein sequences normally unavailable in large quantity, for example, the fibroblast or leukocyte interferons; or their metabolism may be altered so as to induce them to perform some unaccustomed function such as, for example, the conversion of starch to simple sugar.

In order to perform these new protein syntheses, the cells must be 1) made to take up the desired coding sequence and 2) be provided with control sequences which are compatible with the host in operable linkage to the coding sequences. Means must be provided to evaluate the success of both of these requirements. The present invention provides an improved control sequence cassette subject to such evaluation.

### Control Sequences

The dominant selectable marker described herein provided a useful tool to obtain the highly efficient temperature-sensitive control cassette of the invention.

Control sequences are conveniently provided as portable cassettes which could be shuttled between plasmids to precede protein encoding sequences at will. Such portable sequences are, in fact, known in the art. For example, the trp promoter/operator system, including its ribosome binding site and some leader sequence codons, has been described in detail (Goeddel, et al,

### Nucleic Acids Res (1980) 8:4057).

In addition to the convenience of a portable sytem, however, it is also required that the control system be regulatable by control of external parameters. Because bacterial hosts are often called upon to produce proteins which ore not endogenous, premature production of these proteins during the growth phase of the culture may have an adverse effect on the health of the host cells. In order to obtain healthy growth, as well as a good quantitative protein production, it may be necessary to repress the expression of the desired gene during the growth phase of the culture, and then to permit expression after the growth phase has been substantially completed.

The presently available portable promoter systems, while subject to such control, are imperfect in their degree of attainment. For example, the above-mentioned trp promoter is regulated in response to the presence or absence of tryptophan in the medium. The promoter is turned on in the absence of tryptophan, but repressed in its presence. Neither a complete "on" or "off" position is attainable with such a promoter, however. For most bacteria, the indigenous repressor gene does not provide sufficient repressor to interact completely with the desirable higher levels of promoter/coding sequence constructs present on multi-copy plasmids within the cell. Conversely, since many proteins, desired to be synthesized, themselves contain tryptophan, it is not possible to delete tryptophan entirely from the medium when they are to be produced. Even approximate control is troublesome as medium exchange is often required to change the tryptophan level sufficiently.

The λ phage promoter, P_{L}, however, is subject to more finely tuned and convenient control, because the repressor which binds to its operator sequence can be temperature sensitive. The P_{L} operator is repressed at low temperature by repressor proteins such as cI857 which ore synthesized by appropriate mutant host cells. However, at higher temperatures, this repressor protein is deactivated, and the promoter is switched on. Thus, simply by raising the temperature of the culture, the synthesis of the desired protein sequence can be turned on. The P_{L} promoter in its native environment operates to synthesize, among other things, an "N-gene" protein whose translation is controlled by an N_{RBS} ribosome binding site. In the phage, the N-gene is the first coding sequence in the polycistronic message under the control of the P_{L} promoter. P_{L} promoter alone, and p_{L} promoter on a sequence with the N_{RBS} (which is not functionally utilized) have, indeed, been used previously to control the expression of genes encoding E. coli or foreign proteins in bacterial systems. See Shimatake, et al, Nature (1981) 292:128; Remaut, E., et al, Nucleic Acids Res (1983) 11:4677; Remaut, E., et al, Gene (1983) 22:103. However, this control system has not been packaged into a readily transposable segment which can be shuttled from one expression vector to another with ease. In fact, disclosures of the location of the N_{RBS} sequence in E. coli lambda phage, and therefore the composition of the disclosed sequence, have been grossly in error (Scherer, G. F. E., et al, Nucleic Acids Res (1980) 8:3895 at p 3898).

By employing the selectable G418 resistance marker, the exquisitely regulatable P_{L}N_{RBS} control sequence his been packaged into a conveniently excised segment in suitable source vectors so that it can be used either by placing the P_{L}N_{RBS} sequences in front of an ATG start codon, or the P_{L}N_{RBS} can bring with it a blunt 3' end ATG for ligation onto N-terminal blunt-ended coding sequence.

### Disclosure of the Invention

The present invention provides a "cassette" DNA sequence having convenient restriction sites at both 5' and 3' ends to provide ready portability, and having control elements for protein synthesis which are easily regulated by simple manipulations. The sequence contains the P_{L} promoter from lambda phage as well as the N_{RBS} (ribosome binding site for N-gene), immediately preceding a restriction site.

Thus in one aspect, the invention relates to a DNA sequence comprising the P_{L} promoter operably linked to the N_{RBS} having a Hind III restriction cleavage site within 6 bp downstream of the N_{RBS}, and to vectors containing the sequence. The resulting vectors may, of course, no longer contain the restriction cleavage site. Cells or cell cultures transformed with such vectors and progeny thereof are also an aspect of the invention.

In another aspect, the invention relates to a DNA sequence comprising the P_{L} promoter operably linked to the N_{RBS} and to an ATG start codon, the sequence having a restriction site within 6 bp downstream of the ATG, as well as to vectors containing this sequence, where, again the cleavage site may now be absent.

In other aspects, the invention relates to a method for producing a desired protein in recombinant host cells which comprises culturing the above-mentioned transformants and recovering the protein produced. In other aspects, the invention is directed to a method of constructing expression vectors which comprises ligating the P_{L}N_{RBS} cassettes of the invention into operable position with respect to the desired coding sequences.

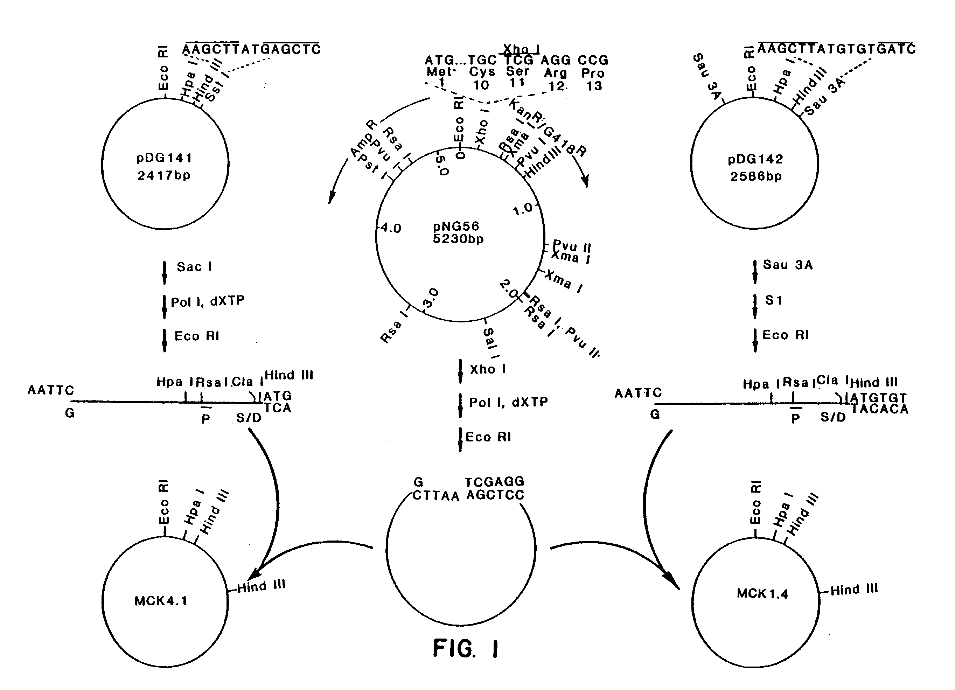

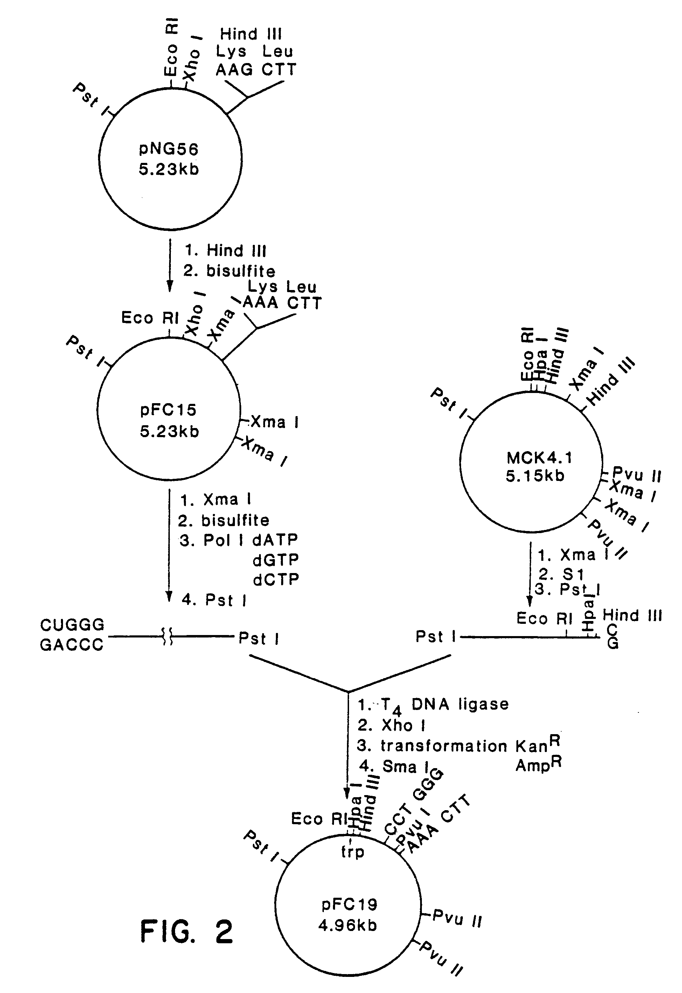

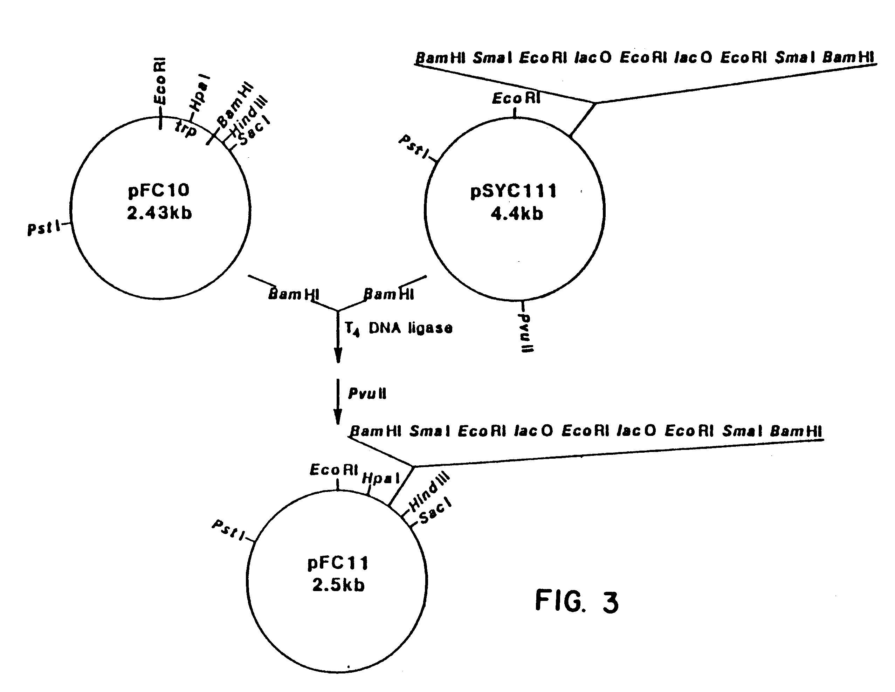

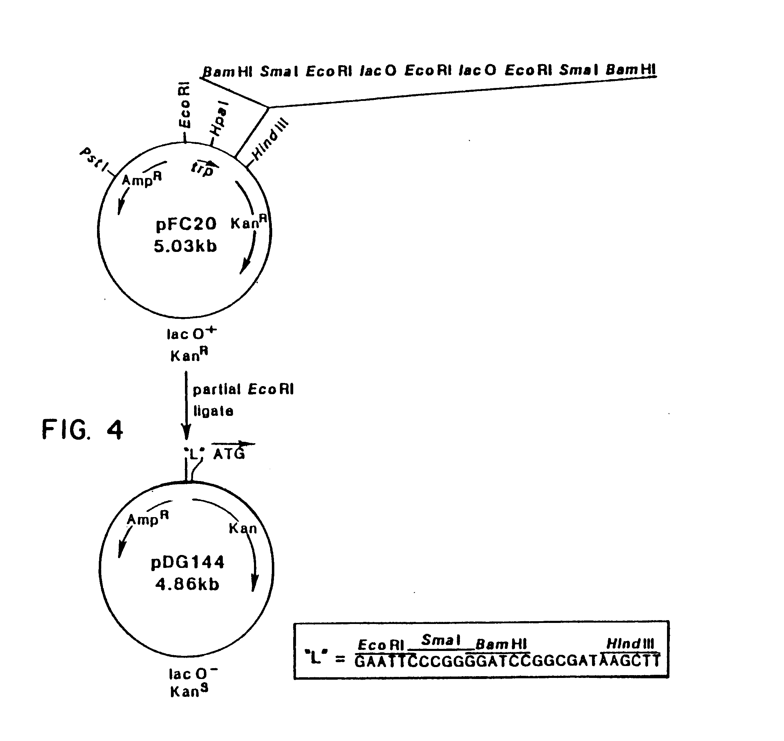

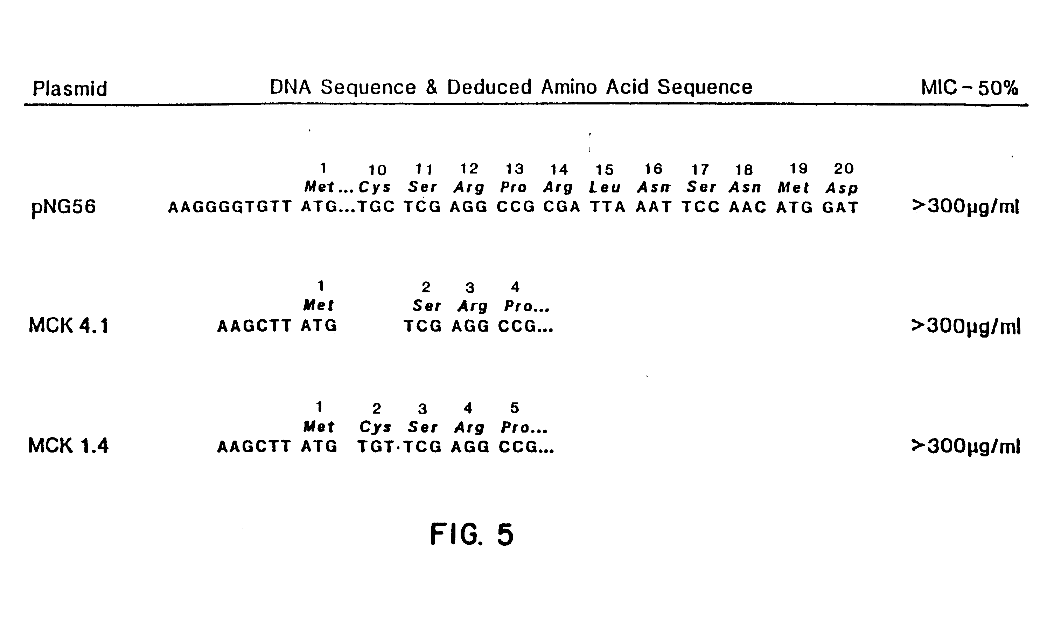

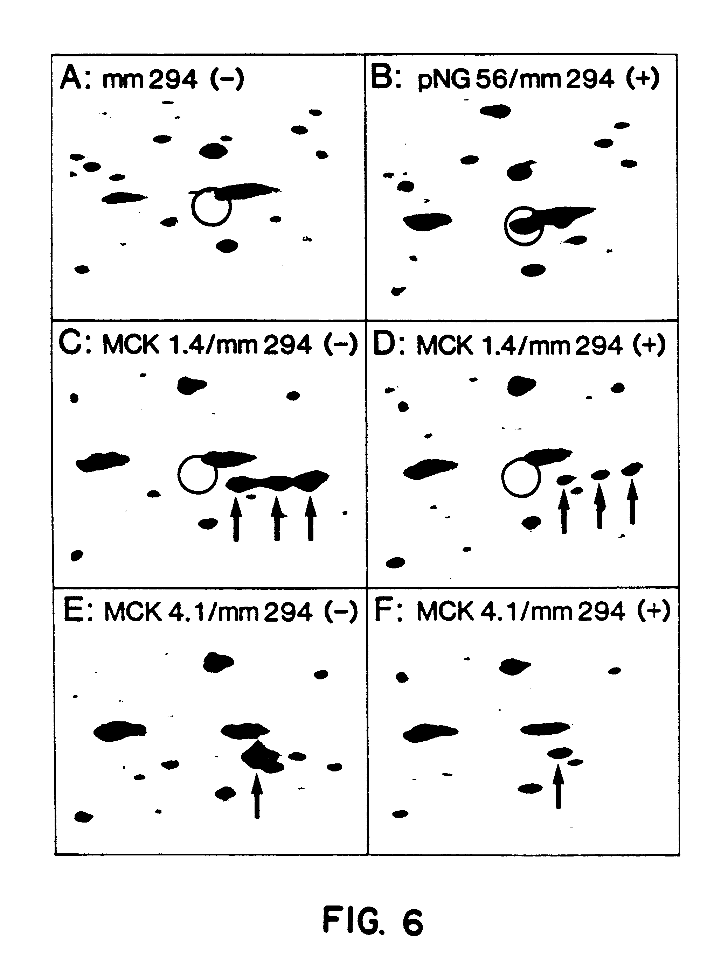

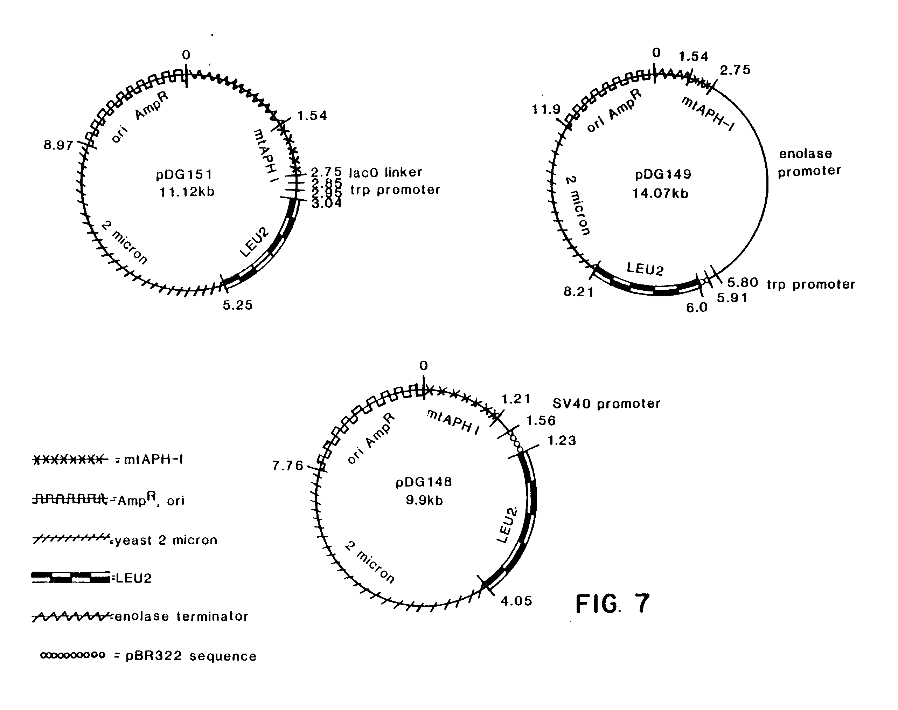

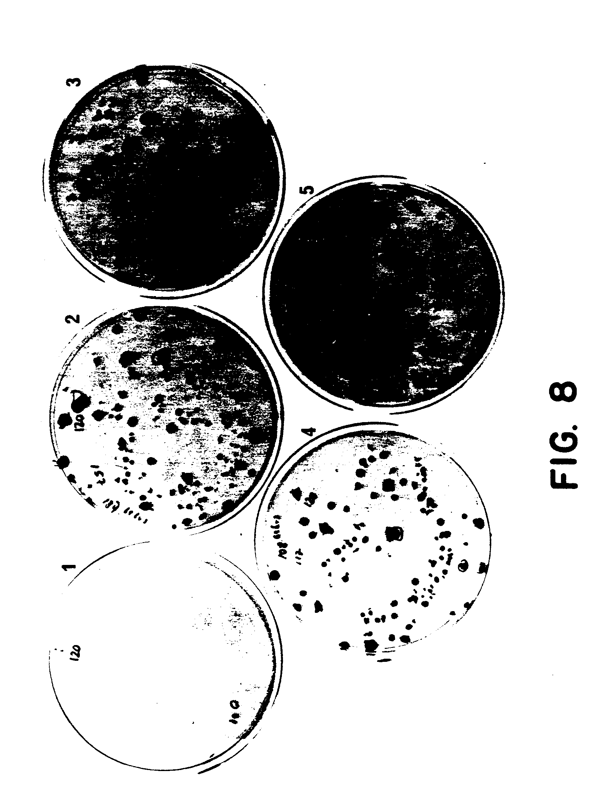

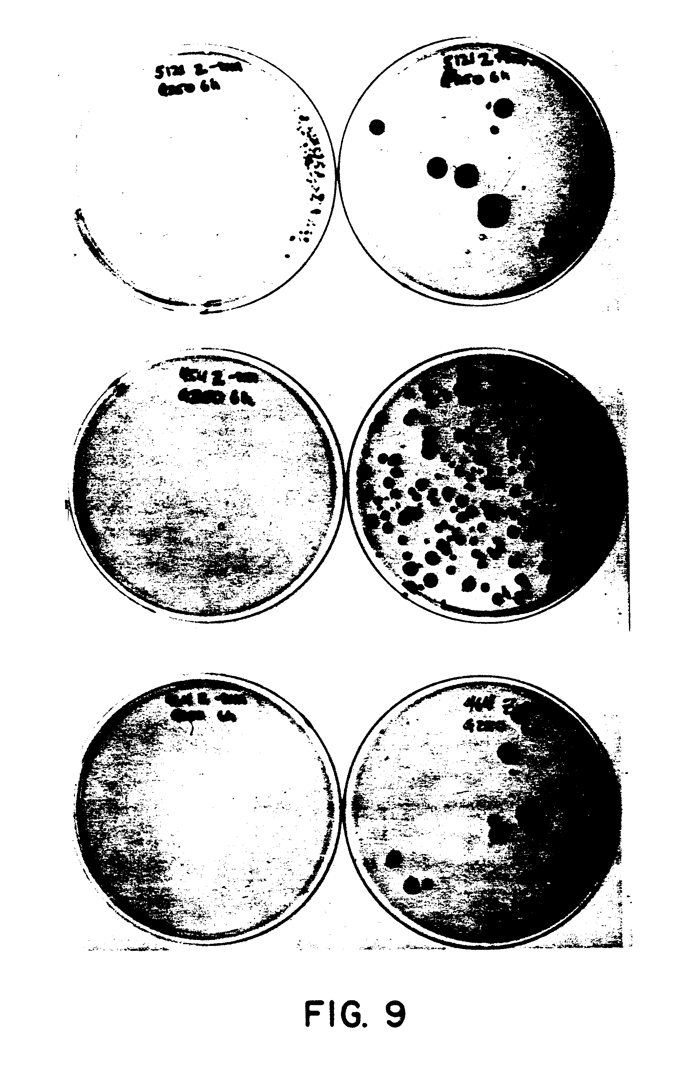

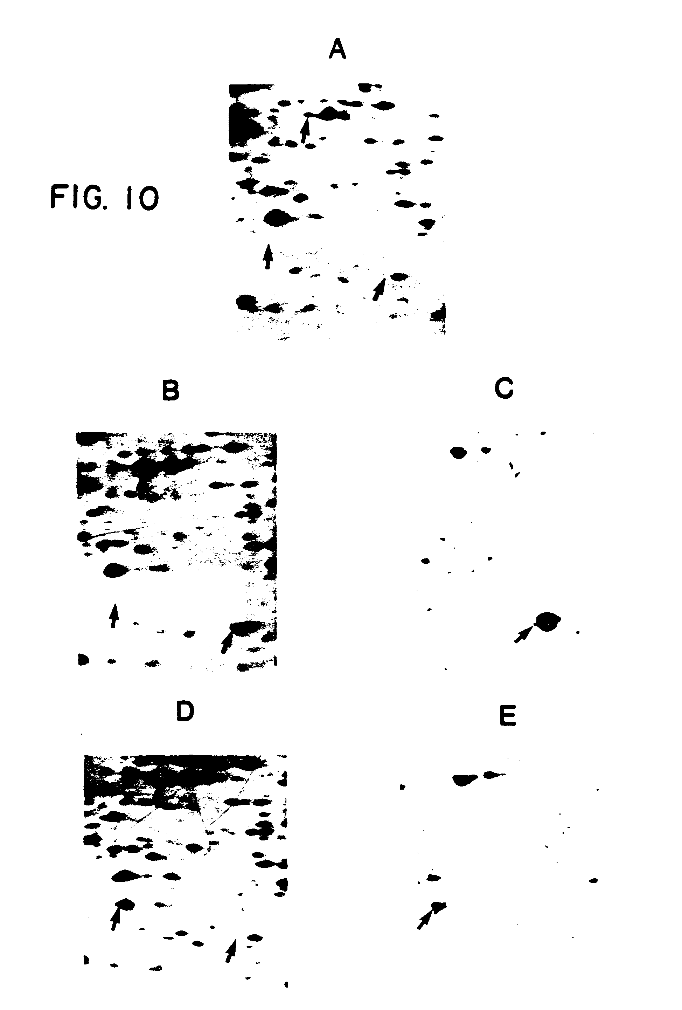

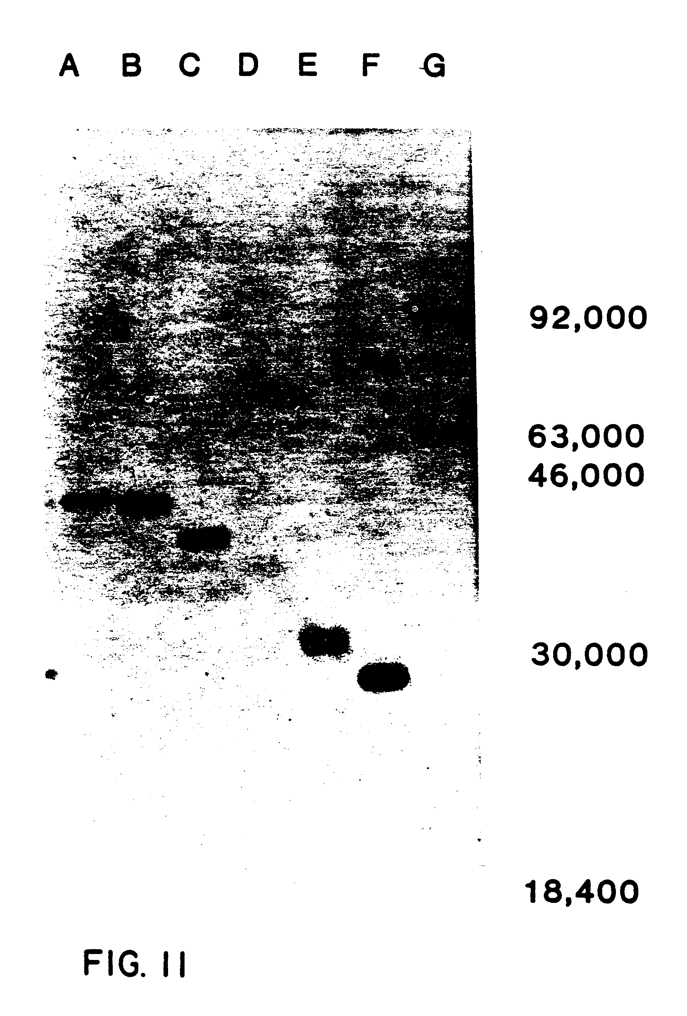

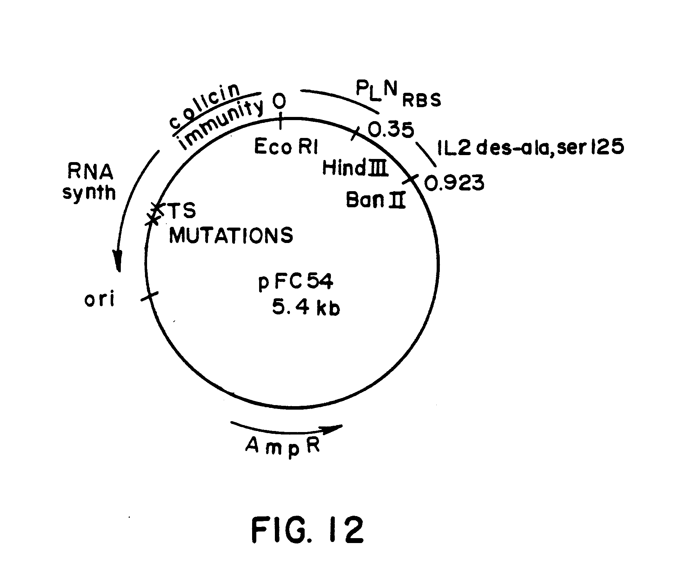

### Brief Description of the Drawing

The accompanying Figure shows the construction of pFC54.

### Modes for Carrying Out the Invention

### A. Definitions

"P_{L}N_{RBS} cassette" refers to a DNA sequence which contains the P_{L} promoter sequence operably linked to N_{RBS}, which cassette is bounded by restriction sites upstream of the P_{L} promoter sequence and downstream from the N_{RBS} sequence. While the exact location of the upstream restriction site is not of importance, the location of the downstream site must be close enough to permit operable juxtaposition to an ATG "start" codon. Thus, the downstream restriction site must permit cleavage within 6 bp of the last base pair of the RBS so that the entire cassette can be conveniently inserted within workable distance of an ATG start codon for the desired protein.

"P_{L}N_{RBS} ATG-cassette" refers to a DNA sequence which contains a P_{L} promoter operably linked to the N_{RBS} coding sequence and to an ATG start codon. The cassette contains a downstream restriction site which permits cleavage within 6 bp 3' of the G of the ATG start codon. This permits convenient ligation of the blunted cassette into reading frame with a desired coding sequence.

Cleavage "within 'n' bp" of a given location requires that at least one of the two strands be so cleaved. As is known, most restriction enzymes catalyze cleavage in a jagged pattern, the cleavage site in one strand being separated by several bp from the cleavage site in the complementary strand. To fit the herein definition, only one of such sites needs to be within the required "h" bp.

The vectors containing the cassette of the invention may or may not contain a cleavage site as described above. Depending on the protocol for ligation of the cassette into a recipient vector, the site may or may not be lost. "N_{RBS}" and "N_{RBS} corresponding sequence' or "ribosome binding site" and "ribosome binding site corresponding sequence" are also used interchangeably in that the embodiment intended will be clear from the context. Thus, when N_{RBS} is used to describe a portion of DNA, clearly "N_{RBS} corresponding sequence" is meant.

"IL-2" refers to proteins having sequence homology with, and the functionality of, interleukin-2. Proteins having the precise sequence of native IL-2 are included, as well as sequences containing sequence modifications which do not destroy activity. In the illustration set forth below, a modification or "mutein" having a serine residue rather than a cysteine at position 125 is used.

"Operably linked" refers to constructions wherein the components so described are juxtaposed in such a way as to permit them to function in their intended manner vis-a-vis each other. Thus, a promoter operably linked to a coding sequence refers to a promoter which is capable of effecting the transcription of the desired coding sequence.

"Polyadenylation signal" or "terminator" is intended to include whatever is required in these sequences, not to be limited simply to this particular function. This definition is necessitated by the current state of knowledge in the art, wherein the relevance of sequences in addition to the polyadenylation signal is unclear.

"Recombinant host cell" refers to a cell which has been transformed with DNA sequences which have been manipulated by recombinant techniques.

"Cells" and "cell culture" are used interchangeably where the context so permits, and these terms include the progeny of any specific cells referred to. Thus, these terms refer to cells whether separated from or suspended in the medium and whether living or dead. These terms thus include progeny of an original transformed cultures, which progeny may differ by mutation from the parental cells.

### B. General Description

Three vector plasmids which are included in the invention, and which provide a convenient source for the P_{L}N_{RBS} cassette containing a restriction site suitable for insertion of the cassette immediately upstream from an ATG are described. Other plasmids can easily be constructed by moving the cassette to any other host plasmids containing the proper sites. Such sites may be directly inserted into a host plasmid at the location of identical or compatible restriction sites normally available in the host plasmid, or at newly constructed compatible sites which can be engineered by digestion of the host plasmid at available sites and insertion of suitable commercially available linkers. Additional plasmids are described which provide the P_{L}N_{RBS}-ATG cassette, i.e., the cassette contains the ATG start codon operably linked to the ribosome binding site. Other plasmids suitable for cloning this cassette can be constructed using methods known in the art.

### C. Methods Employed

Both cloning and expression vectors for desired sequences were constructed using the below described commonly employed restriction and ligation procedures. Additional plasmids, analogous to those illustrated, can also be constructed using these methods, which are well known in the art, by utilizing alternative replicons, vector fragments, control sequences, coding sequences, polylinkers, and expression cassettes.

In general, the quantity of DNA available can be increased by cloning the desired fragments, i.e., inserting into a suitable cloning vehicle, such as pBR322, transforming and replicating in E. coli, and, optionally, further enhancing through chloramphenicol amplification or by phage replication. For expression, the desired fragments can then be removed from the cloning vectors or phage and ligated to suitable control sequences compatible with the host intended to be employed in the expression of the gene. Such hosts are then transformed with these expression vectors and cultured under conditions which favor stabilization of the plasmid and the safe production of a desired protein fragments. Such conditions might include repression of the controlling promoter until most of log phase has been completed, and then alteration of conditions so as to favor the synthesis of the peptide.

### C.1 Transformations

Transformations in the examples below were performed using the calcium chloride method as described by Cohen, S.N., Proc Natl Acad Sci (USA) (1972) 69:2110. The calcium treatment is used for procaryotes or other cells which contain substantial cell wall barriers.

### C.2 Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM MgCl₂, 6 mM DTT and 5-10 µM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with S1 nuclease results in hydrolysis of any single-stranded portion.

Synthetic oligonucleotides are prepared by the triester method of Matteucci, et al (J Am Chem Soc (1981) 103:3185-3191). Kinasing of single strands prior to annealing or for labeling is achieved using an excess, e.g., approximately 10 units of polynucleotide kinase to 1 nmole substrate in the presence of 50 mM Tris, pH 7.6, 10 mM MgCl₂_{,} 5 mM dithiothreitol, 1-2 mM ATP, 1.7 pmoles γ³² P ATP (2.9 mCi/mmole), 0.1 mM spermidine, 0.1 mM EDTA.

Ligations are performed in 15-30 1 volumes under the the following standard conditins and temperatures: 20 mM Tris-Cl pH 7.5, 10 mM MgCl₂_{,} 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations (usually employing a 10-30 fold molar excess of linkers) are performed at 1 µM total ends concentration.

In vector construction employing "vector fragments," the vector fragment is commonly treated with bacterial alkaline phosphatase (BAP) in order to remove the 5' phosphate and prevent religation of the vector. BAP digestions are conducted at pH 8 in approximately 150 mM Tris, in the presence of Na⁺ and Mg⁺² using about 1 unit of BAP per µg of vector at 60° for about one hour. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

### C.3 Verification of Construction

In the constructions set forth below, correct ligations for plasmid construction are confirmed by transforming E. coli strain MM294 obtained from E. coli Genetic Stock Center, CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, following chloramphenicol amplification (Clewell, D. B., J Bacteriol (1972) 110:667). The isolated DNA is analyzed by restriction and/or sequenced by the dideoxy method of Sanger, F. et al, Proc Natl Acad Sci (USA) (1977) 74:5463 as further desribed by Messing, et al, Nucleic Acids Res (1981) 9:309, or by the method of Maxam, et al, Methods in Enzymology (1980) 65:499.

### C.4 Hosts

Two host strains were used in cloning and expression of the plasmids set forth below:
For cloning and sequencing, and for expression of construction under control of most bacterial promoters, E. coli strain MM294 (supra), Talmadge, K., et al, Gene (1980) 12:235; Meselson, M., et al, Nature (1968) 217:1110, was used as the host.

However, when expression is under control of the procaryotic P_{L} promoter and N_{RBS} the E. coli strain MC1000 Lambda N₇N₅₃cI857SusP₈₀ as an expression host was used (ATCC 39531, deposited December 21, 1983). This strain is hereinafter referred to as MC1000-39531. This strain contains a lambda prophage which codes for a temperature sensitive CI repressor, which at the permissive temperature (30-32°C) is active. At the non-permissive temperature (36-44°C), the repressor is inactive and transcription from the P_{L} promoter can proceed. It is further characteristic of this strain that at elevated temperatures the prophage fails to induce.

### D. Detailed Description of Preferred Embodiments

### Construction of a Donor Plasmid for a Portable P_{L}N_{RBS} EcoRI/HindIII Pre-ATG Cassette

The mtAPH-I gene was used as a convenient marker for the construction of a P_{L}N_{RBS} cassette. First, a DNA sequence containing the P_{L} λ phage promoter and the ribosome binding site for the N-gene (N_{RBS}) was obtained from a derivative of pKC30 described by Shimatake and Rosenberg, Nature (1981) 292:128. pKC30 contains a 2.34 kb fragment from λ phage cloned into the HindIII/BamHI vector fragment from pBR322. The P_{L} promoter and N_{RBS} occupy a segment in pKC30 between a BglII and HpaI site. The derivative has the BglII site converted to an EcoRI site.

The BglII site immediately preceding the P_{L} promoter was converted into an EcoRI site as follows: pKC30 was digested with BglII, repaired with Klenow and dNTPs, and ligated with T4 ligase to an EcoRI linker (available from New England Biolabs) and transformed into E. coli strain MM294 Lambda⁺. Plasmids were isolated from Amp^{R} Tet^{S} transformants and the desired sequence was confirmed by restriction enzyme analysis. The resulting plasmid, pFC3, was double-digested with PvuI and HpaI to obtain an approximately 540 bp fragment framing the desired sequence. This fragment was partially digested with HinfI and the 424 bp fragment isolated and treated wiih Klenow and dATP, followed by SI nuclease, to generate a blunt-ended fragment with 3' terminal sequence -AGGAGAA where the -AGGAGA portion is the N_{RBS}. This fragment was treated with EcoRI to give a 347 base pair DNA fragment with 5'-EcoRI/HinfI(partial repair, S1 blunt)-3' termini.

To obtain a plasmid containing desired EcoRI/Hind III cassette containing P_{L}N_{RBS}, the resulting fragment was ligated into an EcoRI/Hind III (repaired) cleaved plasmid vector fragment obtained from pDG144. pDG144, deposited 13th January 1984, ATCC No. 39579 is an altered pBR322 containing an intact Amp^{R} gene, and a coding sequence for a protein conferring resistance to kanamycin (Kan^{R}). The Kan^{R} coding sequence is preceded by a synthetic polylinker. Since pDG144 contains neither a promoter nor a ribosome binding site preceding the coding sequence, Kan^{R} is not expressed, and cells harboring pDG144 are sensitive to kanamycin and to structurally similar antibiotics. The polylinker sequence immediately preceding the ATG start codon for the kanamycin gene can be removed by digesting with EcoRI and Hind III and P_{L}N_{RBS} inserted.

Accordingly, pDG144 was digested with HindIII, blunt-ended with Klenow and dNTPs, and then digested with EcoRI. The vector fragment was ligated with the above-prepared EcoRI/HinfI(repaired) fragment and transformed into MC1000-39531. Amp^{R} Kan^{R} colonies were selected, plasmids isolated and the correct sequence construction was verified by restriction analysis and sequencing. One plasmid containing the correct sequence was designated P_{L}Kan.

An alternative vector to provide the promoter-RBS cassette, pFC5, uses the lac-Z fusion flag as a marker for successful construction. pβI-Z15, deposited 13 January 1984, ATCC No. 39578, was prepared by fusing a sequence containing ATG plus 140 bp of β-IFN fused to lac-Z into pBR322. In pβI-Z15, the EcoRI site of pBR322 is retained, and the insert contains a HindIII site immediately preceding the ATG start codon. pβI-Z15 was restricted with HindIII, repaired with Klenow and dNTPs, and then digested with EcoRI. The resulting EcoRI/HindIII(repaired) vector fragment was ligated with the EcoRI/HinfI(repaired) fragment above. The ligation mixture was used to transform MC1000-39531 and Amp^{R} transformants containing the successful construction were identified by ability to grow on lactose minimal plates at 34°C but not at 30°C. (Transformations were plated on X-gal-Amp plates at 30° and 34° and on minimal-lactose plates at 30° and 34°. Transformants with the proper construction are blue on X-gal-Amp plates at both temperatures, but grow on minimal lactose plates only at 34°.) The successful construct was designated pFC5.

pFC5 or pP_{L}Kan and analogous vectors constructed so as to contain this cassette along with an appropriate marker and replicon, may be used to clone and provide a source for the EcoRI/HindIII P_{L}N_{RBS} fragment. The cassette can then conveniently be placed into an expression vector for a desired sequence behind an ATG start codon for the sequence where this codon contains a HindIII site proximately preceding it.

### Construction of Vectors Providing the P_{L}N_{RBS}-ATG Cassette - pP_{L}N_{RBS} ATG and pDG141(P_{L})

The P_{L}N_{RBS} cassette may also be provided with an operably linked ATG start codon by ligation of the des-ATG cassette into a suitable host vector. pBW20, a derivative of pBR322 containing a synthetic sequence providing an ATG start codon an appropriate distance downstream from a HindIII site, can be thus used.

To prepare pBW20, pBR322 was digested with HindIII, repaired with Klenow and the four dNTPs, then digested with PvuII. The vector fragment was then ligated in a standard blunt-end ligation with the self-complementary dodecamer TATGAGCTCATA, which contains a SacI recognition site partially overlapping and downstream from the ATG sequence. The ligation mixture was transformed into E. coli MM294, and correct construction confirmed by isolation of the plasmids and Maxam-Gilbert sequencing. The resulting pertinent sequence in pBW20 is as follows: 
HindIII SacI
- - - - A A G C T T A T G A G C T C A T A C T G - - - -
pBR322 pBR322
To prepare pP_{L}N_{RBS} ATG, pP_{L}Kan was digested with HindIII and EcoRI and ligated to the EcoRI/HindIII digested BAPed vector fragment from pBW20, transformed into E. coli MC1000-39531. Amp^{R} colonies were selected, and the desired vector construction was confirmed by sequencing or restriction enzyme analysis.

One cloned colony containing the correct construction, pP_{L}N_{RBS} ATG, thus provides a source for a P_{L}N_{RBS}-ATG cassette in a host plasmid suitable for insertion of a gene sequence desired to be expressed. The properly restricted host vector containing the cassette can be provided by digestion with SacI, blunt ending with Klenow or SI nuclease, and insertion of the blunt-ended gene.

pDG141, deposited in the ATCC on 24 January 1984; and given accession number 39588, can also provide the ATG for the P_{L}N_{RBS}-ATG cassette. pDG141 contains the same dodecamer insert as pBW20 in front of a trp promoter cassette. Thus, following the procedure of the previous paragraph, but substituting for pBW20, pDG141, the analogous plasmid pDG141(P_{L}) is obtained.

### Use of the P_{L}N_{RBS} Cassette in Expression

The following examples illustrate one aspect of the invention by describing the construction of expression vectors suitable for production of IL-2 and the successful expression of the appropriate coding sequences. IL-2 and its modified forms are members of a class of proteins designated lymphokines which are useful in therapy directed against deviant cell metabolism. Of course, any desired peptide could be analogously produced by providing the appropriate coding sequence in a manner analogous to that illustrated for production of IL-2. Further, rather than the P_{L}N_{RBS} cassette, the P_{L}N_{RBS} ATG cassette could be placed in front of the codons for the desired peptide as described above.

Native IL-2 is a 133 amino acid sequence with an alanine at its N-terminus. For expression in procaryotic systems, the codons of the leader sequence in the native gene are replaced by an ATG, resulting in production of protein having a methionine residue at the N-terminus. In the paragraphs below, a 133 amino acid modified IL-2 sequence with N-terminal methionine, lacking the native N-terminal alanine, and containing serine at position 125 designated herein IL-2 des-ala, ser125 is produced under control of the cassette of the invention. Alternate IL-2 constructions employing this cassette, such as that set forth in paragraph H.3, or constructions involving other forms of IL-2 can, of course, be made.

### Construction of pFC54

pFC54 is an expression vector for a modified form of IL-2 wherein the cysteine at position 125 has been replaced by a serine residue (IL-2 des-ala, ser125). It is constructed using pFC5 as a source of the P_{L}N_{RBS} cassette, pLW46 as a source of the coding sequence, and pCS4 as a source of the high copy number replicon, as follows:
pCS4 (see below) and pFC5 were each digested with EcoRI and HindIII and the digests ligated at 1:2 molar ratio, 60 µg/ml under sticky end conditions. The ligated DNA (150 ng) was used to transform MC1000-39531 to Amp^{R}, and Lac⁻ transformants screened for the desired 5.45 kb plasmid. The correct plasmid, pFC8, contained the desired 346 bp EcoRI/HindIII P_{L}N_{RBS} fragment in place of the 110 bp trp control region of pCS4.

pLW46 contains the coding sequence for a mutein IL-2 (des-ala, ser125) as a unique HindIII/BanII fragment. This plasmid transformed into E. coli MM294 was deposited 26 September 1983 and assigned ATCC no. 39452.

pLW46 was digested to completion with PvuII (to cleave the unwanted fragment), HindIII and BanII. pFC8 was also digested to completion with HindIII and BanII. The digests were mixed (4:1 molar ratio, 50 µg/ml DNA), ligated under sticky end conditions, and the mixture (100 ng DNA) was used to transform MC1000-39531 to Amp^{R}. Successful transformants were screened for the desired 5.4 kb plasmid lacking the PstI site of pLW46, containing a unique XbaI site, a unique HindIII site, and yielding a 526 bp EcoRI/XbaI fragment. The desired plasmid was designated pFC54: Its construction is summarized in the Figure.

pFC54 transformed into E. coli DG95 lambda lysogen was deposited in the CMCC as no. 2015, and was deposited with the ATCC on 4 September 1984 and has accession no. 39831.

### Construction of pCS4

pCS4 was constructed from pCS3, a plasmid containing a temperatue-sensitive high copy number replicon, by replacing the smaller EcoRI/BamHI digest fragment from pCS3 with an EcoRI/XhoII digest fragment which contains the trp promoter/βIFN coding sequence obtained from the plasmid p 1trp3-4-1 which was deposited with ATCC 30 March 1984 and has accession no. ATCC 39646. pCS3 was deposited with ATCC 3 June 1982 and has accession no. ATCC 39142.

### Production of IL-2 des-ala, ser125

pFC54 was transformed into E. coli DG95 lambda lysogen and the transformants were grown and induced as follows: Fresh overnight samples of pFC54/DG95 were inoculated into N8-2 media (per 500 mls: 20 mM NH₄Cl, 44 mM KH₂PO₄_{,} 56.2 mM Na₂HPO₄, 18 mM K₂SO₄, 0.4 mM MgSO₄, 6 M ZnSO₄, 6 µM MnSO₄, 0.2 µM CuSO₄, 0.4% glucose, 0.002% thiamine) supplemented with 5 g/l casamino acid, 5 µg/l glucose, 100 µg/ml ampicillin and 10 µM FeSO₄. The cells were grown at 30°C to an OD₆₈₀ of 0.150, and then induced by raising the temperature. These transformants produced IL-2, des-ala, ser125 as 20% of accumulated total cell protein after 1-2 hr of induction at 40°C. To assess the level of production, 0.5 ml of cells were centrifuged and the pellet was resuspended in 20 µl 3% SDS, 62.5 mM Tris HCl, pH 6.8. Following heating at 95°C for 5 minutes the samples were run on 12.5% SDS polyacrylamide 3% stacking gel. The production level was then assayed by the intensity of Coomassie Blue stained SDS-polyacrylamide gels. The activity of the IL-2 produced was verified using standard assay methods.

### Construction of pFC53

An IL-2 sequence having 134 amino acid residues, differing from that above by the presence of an alanine residue immediately after the methionine encoded by an ATG start codon (the normal N-terminus in the native sequence) can be obtained in a manner exactly analogous to that set forth in the previous illustration. The coding sequence for this protein, designated herein IL-2 ser125 is obtained as a HindIII/BanII fragment containing the coding sequence from pLW55. (pLW55 in MC1000-39531 was deposited with the ATCC 18 November 1983 and given accession number 39516.) The resulting plasmid containing the temperature sensitive high copy number replicon from pCS4 and the P_{L}N_{RBS} cassette of the invention from pFC5 was designated pFC53.

Transformation of pFC53 into E. coli DG95 lambda lysogen resulted in cultures which produced IL-2 ser125.

The following plasmids have been deposited at the American Type Culture Collection, Rockville, Maryland, U.S.A. (ATCC) under the terms of the Budapest Treaty on the International Recognition of the Deposit of Microorganisms for the Purposes of Patent Procedure and Regulations thereunder (Budapest Treaty) and are thus maintained and made available according to the terms of the Budapest Treaty. Availability of such strains is not to be construed as a license to practice the invention in contravention of the rights granted under the authority of any government in accordance with its patent laws.

The deposited plasmids have been assigned the indicated ATCC deposit numbers. The plasmids have also been deposited with the Master Culture Collection (CMCC) of Cetus Corporation, Emeryville, California, U.S.A., the assignee of the present application, and assigned the indicated CMCC deposit numbers:

| Plasmid | CMCC Deposit No. | ATCC No. |
|---|---|---|
| pDG144 | 1960 | 39579 |
| pDG141 | 1966 | 39588 |
| pFC5 | 1935 | 39864 |
| pFC54 | 2015 | 39831 |
| pCS3 | -- | 39142 |
| pBI-Z15 | -- | 39578 |

## Claims

1. A portable, regulatable control cassette for expression of a heterologous protein in procaryotes, which cassette is obtainable from the vector deposited under ATCC39831, or is a functional equivalent thereof, having a first DNA sequence which is the P_{L} promoter operably linked to a second DNA sequence corresponding to the RBS of the N-gene of λ phage upstream of a third DNA sequence having a Hind III restriction site which permits cleavage within 6 bp 3' of the RBS sequence.

2. A cassette of claim 1, wherein said first and second DNA sequences are operably linked to an ATG start codon, all upstream of a third DNA sequence having a restriction site which is not present elsewhere in the cassette and which permits cleavage within 6 bp 3' of the G of the ATG start codon.

3. A vector for the expression of a heterologous protein, which vector comprises a cassette of claim 1 or claim 2 operably linked to a DNA sequence coding for said heterologous protein inserted at said restriction site, wherein the coding sequence is preceded by an included start codon.

4. Vector pFC54 as obtainable from ATCC39831.

5. Cells or cell cultures transformed with a vector of claim 3 or claim 4.

6. Cells or cell cultures transformed with a vector of claim 3, wherein the vector further includes an origin of replication regulated by temperature.

7. Cells or cell cultures of claim 5 or claim 6 wherein the desired coding sequence encodes IL-2 or a mutein thereof.

8. A method of producing a desired protein which comprises culturing the transformed cells of any one of claims 5, 6 or 7, and recovering the desired protein therefrom.

9. A method of producing a vector as defined in claim 3 including ligating a cassette as defined in claim 1 or claim 2 into operable position with respect to the DNA sequence coding for heterologous protein through insertion of said coding sequence at said restriction site.

10. The use of a cassette as defined in claim 1 or claim 2 in the control of expression of a heterologous protein in a procaryote.

## Patentansprüche

1. Übertragbare regulierbare Kontrollkassette für die Expression eines heterologen Proteins in Prokaryonten, wobei die Kassette von einem Vektor erhältlich ist, der unter der Nummer ATCC39831 hinterlegt wurde, oder ein funktionelles Äquivalent davon ist, mit einer ersten DNA-Sequenz, die der P_{L}-Promotor ist, der funktionell mit einer zweiten DNA-Sequenz verknüpft ist, die der RBS des N-Gens des λ-Phagen entspricht, stromaufwärts von einer dritten DNA-Sequenz mit einer Hind III-Restriktionsstelle, die die Spaltung innerhalb von 6 Basenpaaren 3' von der RBS-Sequenz ermöglicht.

2. Kassette nach Anspruch 1, wobei die ersten und zweiten DNA-Sequenzen funktionell mit einem ATG-Startcodon verknüpft sind, die alle stromaufwärts von einer dritten DNA-Sequenz liegen, die eine Restriktionsstelle aufweist, die sonst nirgendwo in der Kassette vorliegt und die die Spaltung innerhalb von 6 Basenpaaren 3' vom G des ATG-Startcodons ermöglicht.

3. Vektor für die Expression eines heterologen Proteins, wobei der Vektor eine Kassette nach Anspruch 1 oder 2 umfaßt, die funktionell verknüpft ist mit einer DNA-Sequenz, die das an der Restriktionsstelle inserierte heterologe Protein codiert, wobei der codierenden Sequenz ein eingeschlossenes Startcodon vorausgeht.

4. Vektor pFC54, erhältlich von ATCC39831.

5. Zellen oder Zellkulturen, die mit einem Vektor nach Anspruch 3 oder 4 transformiert sind.

6. Zellen oder Zellkulturen, die mit einem Vektor nach Anspruch 3 transformiert sind, wobei der Vektor weiterhin einen Replikationsursprung umfaßt, der temperaturreguliert ist.

7. Zellen oder Zellkulturen nach Anspruch 5 oder 6, wobei die gewünschte codierende Sequenz IL-2 oder ein Mutein davon codiert.

8. Verfahren zur Herstellung eines gewünschten Proteins, umfassend die Züchtung der transformierten Zellen nach einem der Ansprüche 5, 6 oder 7, und Gewinnung des gewünschten Proteins daraus.

9. Verfahren zur Herstellung eines Vektors nach Anspruch 3, umfassend die Ligierung einer Kassette nach Anspruch 1 oder 2 in eine funktionelle Position in Bezug auf die DNA-Sequenz, die das heterologe Protein codiert, durch Insertion der codierenden Sequenz an der Restriktionsstelle.

10. Verwendung einer Kassette nach Anspruch 1 oder 2, in der Expressionskontrolle eines heterologen Proteins in einen Prokaryonten.

## Revendications

1. Cassette portable de contrôle pouvant être régulée destinée à l'expression d'une protéine hétérologue dans des organismes procaryotes, laquelle cassette est obtenue à partir du vecteur déposé auprès de l'ATCC sous le numéro 39831, ou est un équivalent fonctionnel de celle-ci, possédant une première séquence d'ADN qui est le promoteur P_{L} lié de manière opérationnelle à une seconde séquence d'ADN correspondant au RBS (site de liaison ribosonal) de gène N du phage λ en amont d'une troisième séquence d'ADN possédant un site de restriction par HindIII qui permet le clivage au sein des 6 pb en 3' de la séquence RBS.

2. Cassette selon la revendication 1, dans laquelle lesdites première et seconde séquences d'ADN sont liées de manière opérationnelle à un codon d'initiation ATG, toutes deux situées en amont d'une troisième séquence d'ADN possédant un site de restriction qui ne se trouve nulle part ailleurs dans la cassette et qui permet le clivage au sein des 6 pb en 3' du résidu G du codon d'initiation ATG.

3. Vecteur destiné à l'expression d'une protéine hétérologue, lequel vecteur comprend une cassette selon la revendication 1 ou 2 liée de manière opérationnelle à une séquence d'ADN codant pour ladite protéine hétérologue insérée audit site de restriction, dans lequel la séquence codante est précédée d'un codon d'initiation inclus.

4. Vecteur pCF54, tel qu'il est disponible auprès de l'ATCC sous le numéro 39831.

5. Cellules ou culture de cellules transformées par un vecteur selon la revendication 3 ou 4.

6. Cellules ou culture de cellules transformées par un vecteur selon la revendication 3 dans lesquelles le vecteur contient également une origine de réplication régulée par la température.

7. Cellules ou culture de cellules transformées par un vecteur selon la revendication 5 ou 6 dans lesquelles la séquence codante désirée code pour IL-2 ou une mutéine d'IL-2.

8. Procédé pour la production d'une protéine désirée qui comprend la culture de cellules transformées selon l'une des revendications 5, 6 ou 7 et la récupération à partir de cette culture de la protéine désirée.

9. Procédé pour la production d'un vecteur comme défini à la revendication 3 incluant la ligature d'une cassette comme défini à la revendication 1 ou 2 dans une position opérationnelle par rapport à la séquence d'ADN codant pour la protéine hétérologue au moyen de l'insertion de ladite séquence codante audit site de restriction.

10. Utilisation d'une cassette comme défini à la revendication 1 ou 2 pour le contrôle de l'expression d'une protéine hétérologue dans un organisme procaryote.
